(19) Europäisches Patentamt
European Patent Office
Office européen des brevets

(11) Veröffentlichungsnummer: **0 433 616 A2**

# (12) EUROPÄISCHE PATENTANMELDUNG

(21) Anmeldenummer: **90120673.0**

(22) Anmeldetag: **29.10.90**

(51) Int. Cl.5: **A01N 43/66**, A01N 43/82, A01N 43/60, A01N 43/78

(30) Priorität: **22.12.89 DE 3942433**

(43) Veröffentlichungstag der Anmeldung: **26.06.91 Patentblatt 91/26**

(84) Benannte Vertragsstaaten: **AT BE DE FR GB LU NL**

(71) Anmelder: **Degussa Aktiengesellschaft**
**Weissfrauenstrasse 9**
**W-6000 Frankfurt am Main 1(DE)**

(72) Erfinder: **Werle, Peter, Dr.**
**Im Börner 43**
**W-6460 Gelnhausen 2(DE)**
Erfinder: **Trageser, Martin**
**Leipziger Strasse 14**
**W-6460 Gelnhausen(DE)**
Erfinder: **Stober, Reinhard, Dr.**
**Bornwiesenweg 22**
**W-6467 Hasselroth 2(DE)**
Erfinder: **Schwarze, Werner, Dr.**
**Lerchesbergring 99**
**W-6000 Frankfurt 70(DE)**

## (54) Verwendung heterocyclischer Thiocyanate als Mikrobiozide und solche Mikrobiozide enthaltende Mittel.

(57) Die Erfindung richtet sich auf die Verwendung der heterocyclischen Thiocyanate
2, 4-Dithiocyanato-6-amino-1,3,5-triazin (I),
3, 5-Dithiocyanato-1,2,4-thiadiazol (II),
2, 5-Dithiocyanato-1,3,4-thiadiazol (III) und
2,3-Dithiocyanatochinoxalin (IV) sowie
2-Thiocyanatobenzthiazol (V) als Mikrobiozide, welche eine hohe Aktivität und ein breites Wirkungsspektrum aufweisen. Die Thiocyanate (I) bis (V) sind insbesondere zur Bekämpfung von Bakterien, Pilzen und Algen geeignet. Die Erfindung betrifft ferner Mikrobiozide der Formeln (I) bis (V) enthaltende Mittel.

EP 0 433 616 A2

# VERWENDUNG HETEROCYCLISCHER THIOCYANATE ALS MIKROBIOZIDE UND SOLCHE MIKROBIOZIDE ENTHALTENDE MITTEL

Die Erfindung richtet sich auf die Verwendung heterocyclischer Thiocyanate der Formeln

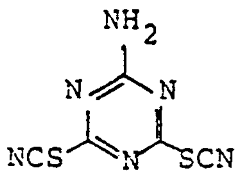

,

(I)

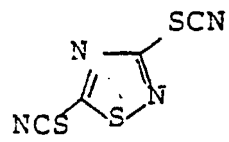

,

(II)

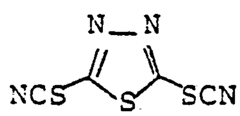

,

(III)

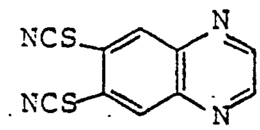

und

(IV)

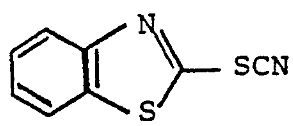

(V)

als Mikrobiozid und solche Mikrobiozide enthaltende Mittel.

Methylenbisthiocyanat der Formel $NCS-CH_2-SCN$ ist seit langem als wirksames Mikrobiozid mit breitem Wirkungsspektrum bekannt, das sich insbesondere für die Wasserbehandlung, vorzugsweise in der Papierindustrie, eignet.

Auch wurden zahlreiche Methylenbisthiocyanat enthaltende, mikrobiozide Mittel vorgeschlagen - vgl. DE-OS 37 18 661 und die dort zitierten Dokumente. Obgleich Methylenbisthiocyanat eine hohe mikrobiozide Wirksamkeit aufweist, sind höhere Homologe, etwa Ethylenbisthiocyanat, um Größenordnungen schwächer wirksam.

Es hat nicht an Versuchen gefehlt, weitere mikrobiozid wirksame Stoffe mit Thiocyanatsubstituenten aufzufinden. So weisen heterocyclische Thiocyanate mit einer s-Triazinstruktur - vgl. DE-PS 10 70 636 der allgemeinen Formel

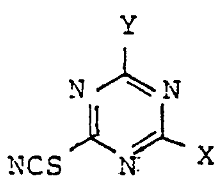

,

worin X eine Thiocyanato-, Alkyl-, Alkylamino- oder Dialkylaminogruppe und Y eine Alkyl-, Alkylamino- oder Dialkylaminogruppe bedeuten, zwar eine gewisse Wirksamkeit gegen Mikroorganismen auf, aber die Aktivität und die Breite des Wirkungsspektrums reichen bei weitem nicht an die Wirksamkeit von Methylenbisthiocyanat heran. Ähnliches gilt für viele andere aromatische und heteroaromatische Verbindungen mit einem oder zwei Thiocyanatsubstituenten.

Zwar sind in 2,5-Stellung substituierte 1,3,4-Thiadiazole als Mikrobiozide bekannt geworden, bei den Substituenten handelt es sich aber nicht um Thiocyanatogruppen, sondern um Halogenalkyl-, Alkylsulfinyl- bzw. Alkylsulfonylgruppen - vgl. DE-OS 37 22 320.

Es bestand die Aufgabe, heterocyclische Thiocyanate aufzufinden, welche als Mikrobiozide mit hoher Aktivität und breitem Wirkungsspektrum Verwendung finden können.

Gefunden wurde die Verwendung heterocyclischer Thiocyanate der Formeln

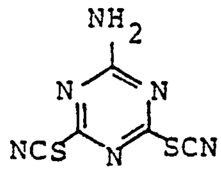 (I),

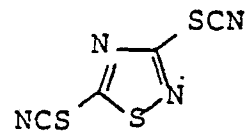 (II),

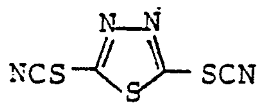 (III),

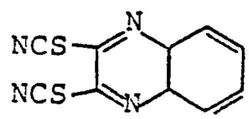 (IV) und

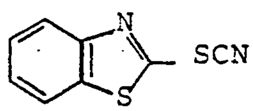 (V)

als Mikrobiozide.

Bei den genannten Verbindungen handelt es sich um
2,4-Dithiocyanato-6-amino-1,3,5,-triazin (I),
3,5-Dithiocyanato-1,2,4-thiadiazol (II),
2,5-Dithiocyanato-1,3,4-thiadiazol (III),
2,3-Dithiocyanato-chinoxalin (IV) und
2-Thiocyanato-benzthiazol (V).

Gefunden wurden ferner mikrobiozide Mittel, welche dadurch gekennzeichnet sind, daß sie mindestens eines der mikrobiozid wirksamen heterocyclischen Thiocyanate der Formeln (I) bis (V) des Anspruchs 1 in einem wäßrigen oder organischen Trägerstoff gelöst oder suspendiert enthalten.

Es konnte nicht erwartet werden, daß die Thiocyanate der Formeln (I) bis (V) sowohl eine hohe mikrobiozide Aktivität gegen einzelne Mikroorganismen, wie Bakterien, Pilzen und Algen, als auch ein breites Wirkungsspektrum aufweisen.

Die heterocyclischen Thiocyanate (I) bis (IV) lassen sich in ähnlicher Weise herstellen wie 2-Thiocyanatobenzthiazol (V) - vgl. DE-AS 11 95 743. Die den Thiocyanaten (I) bis (V) zugrundeliegenden Mercaptoverbindungen werden in Form eines wasserlöslichen Salzes, vorzugsweise Alkalisalzes, mit Chlorcyan umgesetzt, und nach beendeter Umsetzung werden die Thiocyanate (I) bis (V) von dem Chlorid, vorzugsweise Alkalichlorid, abgetrennt. Die Umsetzung kann in einem wäßrigen oder wäßrigorganischen Reaktionsmedium, vorzugsweise in Wasser als alleinigem Lösungsmittel für die Reaktionspartner, durchgeführt werden. Das Reaktionsmedium kann eine homogene Phase oder ein Zweiphasensystem (z.B. Wasser/ organisches Lösungsmittel) bilden. Im allgemeinen erfolgt die Umsetzung bei Temperaturen im Bereich von 0 bis 60 °C, vorzugsweise 5 bis 30 °C, wobei, sofern erforderlich, auch ein mäßiger Überdruck aufrechterhalten werden kann, um Chlorcyanverluste zu vermeiden. Gemäß einer besonders bevorzugten Ausführungsform wird eine wäßrige Lösung des Mercaptids mit einer wäßrigen Lösung des Chlorcyans umgesetzt, wobei das heterocyclische Thiocyanat ausfällt und anschließend durch Fest-Flüssig-Phasentrennung von der das Chlorid enthaltenden wäßrigen Phase abgetrennt wird - siehe Beispiele 1 bis 4. Die Herstellung von 3. 5-Dithiocyanato-1,2,4-thiadiazol aus der entsprechenden Mercaptoverbindung und Bromcyan ist aus Svensk

Kem. Tid 56, S. 207-209 (1944) bekannt.

Die Bestimmung der mikrobioziden Wirkung kann in Anlehnung an den sogenannten Time-Kill-Test durchgeführt werden (vgl. American Petrol Institut, RP 38 2nd ed., Dec. 1985). Dieser Test liegt den Untersuchungen des Beispiels 5 zugrunde.

Die heterocyclischen Thiocyanate der Formeln (I) bis (V) können entweder direkt oder in Form von mikrobioziden Mitteln zur Abtötung von Mikroorganismen verwendet werden. Zur Herstellung der mikrobioziden Mittel wird mindestens eines der erfindungsgemäßen Mikrobiozide der Formeln (I) bis (V) in einem wäßrigen oder organischen flüssigen Trägerstoff, gelöst oder suspendiert. Solche Mittel können zusätzlich ein oder mehrere Biozide aus der gleichen Stoffklasse, also der Thiocyanate, oder anderen mit Thiocyanaten verträglichen Stoffklassen, wie beispielsweise der quaternären Ammoniumverbindungen, Dithiocarbaminsäureester, Aldehyde, 1 ,3. 5-Tris(2-hydroxyethyl)hexahydro-s-triazin, enthalten. Als weitere Bestandteile können die mikrobioziden Mittel systemverträgliche Hilfsstoffe enthalten, etwa dipolare organische Lösungsvermittler im Falle von Mikrobiozid-Lösungen oder Suspensionshilfsmittel im Falle von Mikrobiozid-Suspensionen, beispielsweise Xanthan oder andere Polysaccharide und/oder Polyvinylalkohol sowie Polyvinylpyrrolidon. Zur Herstellung der mikrobioziden Mittel werden die festen und flüssigen Bestandteile in an sich bekannter Weise unter Rühren und ggf. mäßiger Temperaturerhöhung in eine Lösung bzw. eine Suspension überführt. Die Mittel können vorzugsweise in der Wasserbehandlung zur Bekämpfung von Bakterien, Pilzen und Algen, aber auch zur Bekämpfung dieser Keime in technischen Materialen, wie z. B. Kunstharzdispersionen, eingesetzt werden. Die algizide Wirkung der heterocyclischen Thiocyanate wurde an Chlorella vulgaris nachgewiesen.

Die nachfolgenden Beispiele verdeutlichen die Erfindung.

Beispiel 1: Herstellung von 2, 5-Dithiocyanato-1,3,4-thiadiazol (III)

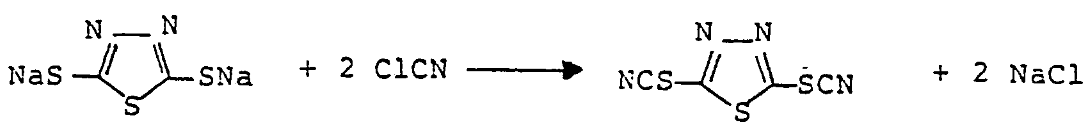

184,5 g (3,0 Mol) Chlorcyan werden in 800 ml Wasser von 5-10 °C in einem Kolben vorgelegt; dazu läßt man eine konz. wäßrige Lösung von 290 g (1,5 Mol) 2, 5-Dimercapto-1,3,4-thiadiazol-Dinatriumsalz zutropfen. Man rührt 30 Min. bei etwa 20 °C nach, saugt das ausgefallene schwach beige gefärbte Produkt ab, wäscht mit Wasser und trocknet. Ausbeute: 295 g (98 % d. Th. ) , Schmelzpunkt 162 °C.

| | | | | |
|---|---|---|---|---|
| Analyse: | Berechnet | C 24,0 | N 28,0 | S 48,0 |
| | Gefunden | C 24,2 | N 28,3 | S 47,5 |

Beispiel 2: Herstellung von 3,5-Dithiocyanato-1,2,4-thiadiazol (II)

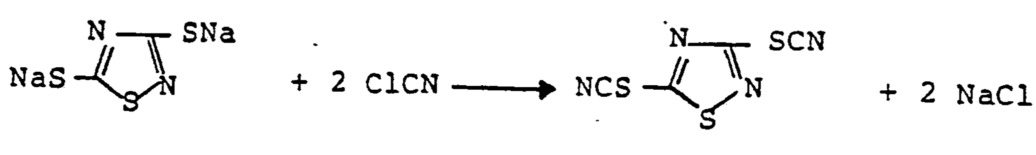

Die Synthese erfolgt analog der unter Beispiel 1 gegebenen Vorschrift.

| | | | | |
|---|---|---|---|---|
| Analyse: | Berechnet | C 24,0 | N 28,0 | S 48,0 |
| | Gefunden | C 23,8 | N 27,5 | S 48,4 |

Beispiel 3: Herstellung von 2,4-Dithiocyanato-6-amino-1,3,5-triazin (I)

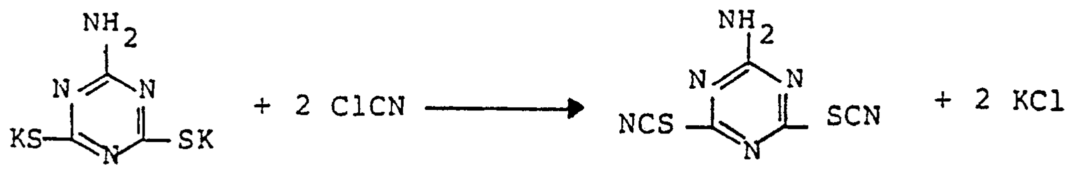

123 g (2,0 Mol) Chlorcyan werden in 500 ml Wasser in einem Kolben vorgelegt, bei 5-10 °c 236 g (1 ,0 Mol) Dikaliumdithioammelid in wäßriger Lösung zugetropft und 45 Min. bei 20 °C nachgerührt. Das ausgefallene Produkt wird abgesaugt, mit Wasser chloridfrei gewaschen und getrocknet. Farbloses Pulver. Ausbeute 195 g (93 % d. Th.)

| | | | | | |
|---|---|---|---|---|---|
| Analyse: | Berechnet | C 28,6 | H 0,96 | N 40,0 | S 30,4 |
| | Gefunden | C 28,5 | H 0,84 | N 39,8 | S 28,5 |

Beispiel 4: Herstellung von 2,3-Dithiocyanat ochinoxalin (IV)

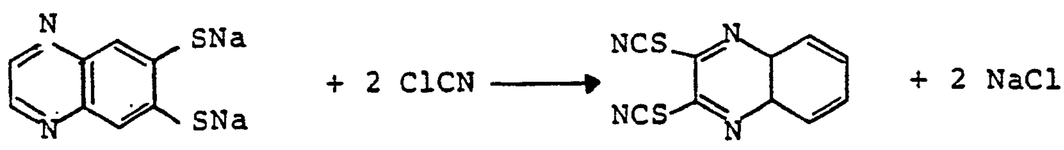

12,3 g (0,2 Mol) Chlorcyan werden in 50 ml Wasser vorgelegt und bei 5-10 °C 23,8 g (0,1 Mol) 2,3-Dimercaptochinoxalin-Dinatriumsalz zugetropft. Man rührt 30 Min. bei 20-30 °C nach, filtriert und trocknet den Rückstand. Schwach gefärbtes Pulver. Ausbeute 21,2 g (87 % d. Th.)

| | | | | | |
|---|---|---|---|---|---|
| Analyse: | Berechnet | C 49,2 | H 1,65 | N 22,9 | S 26,25 |
| | Gefunden | C 50,0 | H 1,30 | N 22,8 | S 25,0 |

Beispiel 5: Bestimmung der mikrobioziden Wirkung im Time-Kill-Test

Bei diesem in Anlehnung an die Empfehlungen des American Petroleums Instituts (API, RP 38 2nd ed., Dec. 1965) durchgeführten Test wird eine hoch angereicherte Keimsuspension (Keimzahl $10^6$-$10^8$) mit der gewünschten Menge Biozid versetzt und 24 h bei 25 °C bebrütet. Anschließend wird eine geometrische Verdünnungsreihe bis 6 durchgeführt; je 1 ml wird mit 10 ml Nähragar auf Platten vermischt und 48 h bei 37 °C bebrütet. Aus der Auszählung der Kolonien wird die Abtötungsrate berechnet.

$$\text{Abtötungsrate in \%} = 100 - \frac{\text{Keimzahl der Probe} \cdot 100}{\text{Keimzahl der Nullprobe}}$$

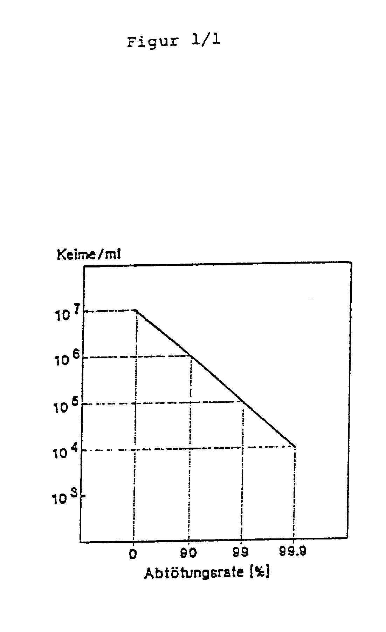

Die Figur 1/1 zeigt das Verhältnis von Keimzahl zur Abtötungsrate. Hieraus wird deutlich, daß auch eine von 99,9 % auf 100 % gesteigerte Abtötungsrate einer bedeutenden Wirkungsverbesserung entspricht.

Der Tabelle ist die biozide Wirksamkeit der erfindungsgemäßen heterocyclischen Thiocyanate der Formeln (I) bis (V) gegen verschiedene Keime aus der Gruppe Pilze und Bakterien zu entnehmen. Angegeben ist die Abtötungsrate gegenüber den Testkeimen, wobei die Anwendungskonzentration der Biozide 10 bis 50 ppm betrug. Die Biozide wurden als Lösung in Dimethylformamid der angereicherten Keimsuspension (40 ml), welche 8,5 g NaCl/l enthielt, zugesetzt.

| Keim | Konz. (ppm) | Heterocyclisches Thiocyanat gemäß Formel | | | | |
|---|---|---|---|---|---|---|
| | | (III) | (II) | (I) | (IV) | (V) |
| Alcaligenes faecalis | 25 | | | | | 99,9 |
| | 10 | 99,9 | 99,3 | 98,4 | | |
| Aspergillus niger | 50 | 99,9 | 99,3 | 99,9 | 99,9 | 99,9 |
| Bacillus subtilis | 25 | 99,9 | 99,9 | | | 99,9 |
| Candida albicans | 25 | | | | | 100,0 |
| | 10 | 99,9 | 100,0 | 99,9 | | |
| Escherichia coli | 25 | | 99,9 | | 98,6 | 99,8 |
| | 10 | 99,9 | | 99,9 | | |
| Klebsiella pneumoniae | 25 | | | 100,0 | | |
| | 10 | | 99,9 | | | 99,9 |
| | 5 | 99,9 | | | | |
| Penicillium funiculosum | 50 | | 100,0 | 99,9 | | |
| | 25 | 99,9 | | | | 99,9 |
| Pseudomonas aeruginosa | 25 | | 92,3 | | | 99,6 |
| | 10 | 99,9 | | | | |
| Sacharomyces cerevisiae | 25 | 100,0 | | | | 100,0 |
| Serratia mercescens | 25 | | | 99,9 | | 99,9 |
| | 10 | 99,9 | 100,0 | | | |
| Staphylococcus aureus | 25 | 99,9 | 99,9 | 99,9 | 99,9 | |

Beispiel 6

Die Bestimmung der algiziden Wirkung der erfindungsgemäßen Thiocyanate (I) bis (V) erfolgte durch Behandlung einer Chlorella vulgaris enthaltenden Testsuspension. Die Testsuspension wurde erhalten durch Zugabe von 1 ml standardisierter Algensuspension in 200 ml Nährlösung und 10tägiges Rühren und 12 Std./ Tag Beleuchtung. Nach Zugabe des zu prüfenden Wirkstoffs zur Testsuspenion und weiterem Rühren verschwindet trotzt Aufrechterhaltung der Bestrahlung (12 Stunden pro Tag) der Testsuspension die grüne Farbe des Chlorophylls innerhalb der in der Tabelle angegebenen Zeit; während sich die Farbe der wirkstofffreien Vergleichsprobe vertieft!

| Thiocyanat (gemäß Formel) | Anwendungs-konzentration (ppm) | Dauer bis zum Entfärben (Tage) |
|---|---|---|
| I | 10 | 6 |
| | 5 | 9 |
| II | 15 | 5 |
| III | 10 | 6 |
| | 5 | 9 |
| IV | 10 | 6 |
| V | 10 | 6 |

## Ansprüche

1. Verwendung heterocyclischer Thiocyanate der Formeln

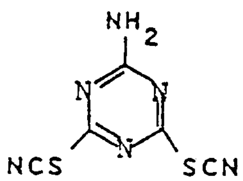

(I),

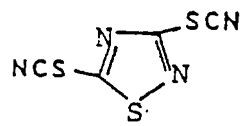

(II),

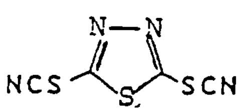

(III) und

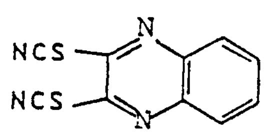

(IV)

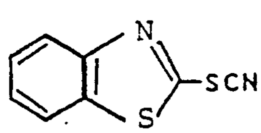

(V)

als Mikrobiozid.

2. Mikrobiozide Mittel.

dadurch gekennzeichnet,
daß sie mindestens eines der mikrobiozid wirksamen heterocyclischen Thiocyanate der Formeln (I) bis (V) des Anspruchs 1 in einem wäßrigen oder organischen Trägerstoff gelöst oder suspendiert enthalten.

Figur 1/1